# EUROPEAN PATENT APPLICATION

(11) **EP 3 751 509 A1**
(43) Date of publication of application: **16.12.2020**
(21) Application number: 19750976.3
(22) Date of filing: 07.02.2019
(51) Int. Cl.: G06T 5/00, A61B 3/10, H04N 1/409

(54) **IMAGE PROCESSING DEVICE, IMAGE PROCESSING METHOD, AND IMAGE PROCESSING PROGRAM**

(30) Priority: 08.02.2018 JP 2018021452
(71) Applicant: KOWA COMPANY, LTD., Naka-ku Nagoya-shi Aichi 460-8625 (JP)
(72) Inventor: YABUSAKI Katsumi, Higashimurayama-shi, Tokyo 189-0022 (JP)
(74) Representative: LKGLOBAL Lorenz & Kopf PartG mbB Patentanwälte
(86) International application number: PCT/JP2019/004337
(87) International publication number: WO 2019/156140

(57) **Abstract**

The image processing device (20) according to the present invention includes an alignment means (31) that arranges a pixel of interest and a plurality of adjacent pixels located around the pixel of interest in order of respective magnitudes of luminance values, a determination means (32) that determines whether the pixel of interest is within a predetermined range intermediate in the pixel of interest and the plurality of adjacent pixels sorted by the alignment means (31), and a substitution means (33) that, when the pixel of interest is not within the predetermined range, substitutes the luminance value of the pixel of interest on the basis of the luminance value of a pixel within the predetermined range.

## Description

### [Technical Field]

The present invention relates to an image processing device, an image processing method, and an image processing program for processing images containing noises to generate images with reduced noises.

### [Background Art]

An image with a poor signal-to-noise ratio (S/N) contains a large amount of noises composed of white points and black points. These noises may lead to incorrect diagnosis/analysis results or cause variations in the obtained results, for example, in the course of image processing that is performed to diagnose/analyze images to obtain some information. Particularly in the medical field, when a two-dimensional image or a tomographic image of an observation site is obtained to perform image diagnosis, the structure of interest which is considered to be abnormal is very small, unclear, or narrow in width. In such a case, a noise point may be erroneously extracted as the target structure of interest, or a noise component may divide the structure of interest, thereby causing an originally single structure to be recognized as a plurality of structures. Thus, how to remove noises is a very important issue in the course of image processing.

Conventionally, as processes for reducing noises in a single image, some methods have been widely used, including a method of focusing on pixels around the pixel of interest and taking an average value or a median value. This is because such processes reduce the contribution of white points and black points, and as a result, noises that largely deviate from the average value of the region are removed. In addition, in the field of processing a plurality of successive images, such as the field of handling tomographic images of an ocular fundus, as disclosed in Patent Document 1, for example, a method has been employed in which noises are reduced by taking a moving average using, as the above, the average value or median value of pixels in two or more adjacent images.

### [Prior Art Documents]

### [Patent Documents]

[Patent Document 1] WO2014/112611A1

### [Summary of the Invention]

### [Problems to be solved by the Invention]

In these conventional methods, however, the peripheral information around the pixel of interest in the image is averaged without exception; therefore, the outline of a structure in the image may be blurred or the luminance of the structure may be deteriorated. Thus, the conventional methods have a problem in that not only the contrast between the structure of interest and the background may be lowered, but also the features themselves, such as the shape, brightness, and color, of the structure may be impaired.

Moreover, in the field of handling tomographic images, in addition to the problem of impairing the features of the structure of interest as described above, the following problem may arise. That is, in the method of taking the moving average of successive tomographic images, the pixels determined to be noises can be easily removed by averaging the pixels of a plurality of images, and smooth successive tomographic images can be generated, but by performing the averaging process on the successive images, structures that are not included in the tomographic image of interest but are included in the tomographic images before and after the tomographic image of interest may be reflected in the tomographic image of interest after the processing, which may be problematic.

The present invention has been made in consideration of the above, and objects of the present invention include providing an image processing device, an image processing method, and an image processing program that are able to reduce noises in an image while maintaining the features of structures in the image.

### [Means for solving the Problems]

To achieve the above objects, first, the present invention provides an image processing device comprising: an alignment means that arranges a pixel of interest and a plurality of adjacent pixels located around the pixel of interest in order of respective magnitudes of luminance values; a determination means that determines whether the pixel of interest is within a predetermined range intermediate in the pixel of interest and the plurality of adjacent pixels sorted by the alignment means; and a substitution means that, when the pixel of interest is not within the predetermined range, substitutes the luminance value of the pixel of interest on the basis of the luminance value of a pixel within the predetermined range (Invention 1).

According to the above invention (Invention 1), rather than simply averaging the luminance value of the pixel of interest together with the luminance values of peripheral pixels (adjacent pixels located around the pixel of interest), the pixel of interest and the peripheral pixels are sorted in order of the magnitudes of luminance values, and when the pixel of interest is included in the predetermined intermediate range, no action is performed, while only when the pixel of interest is not included in the predetermined range, the luminance value of the pixel of interest is substituted based on the luminance value of a pixel within the predetermined range; therefore, only the noises in an image can be removed. As a result, the process of reducing noises can be performed without impairing the features such as the shape, brightness, and color of a characteristic structure captured in the image.

In the above invention (Invention 1), the predetermined range may preferably be set so as to include the same number of pixels before and after a pixel located at the center of the pixel of interest and the plurality of adjacent pixels sorted by the alignment means (Invention 2).

In the above invention (Invention 1, 2), the substitution means may preferably substitute the luminance value of the pixel of interest with an average value of luminance values of pixels within the predetermined range (Invention 3).

In the above invention (Invention 1 to 3), the pixel of interest may exist in an image that is one tomographic image of a plurality of successive tomographic images, and the plurality of adjacent pixels may be a plurality of adjacent pixels that are spatially located around the pixel of interest in the one tomographic image and tomographic images before and after the one tomographic image (Invention 4).

According to the above invention (Invention 4), even when successive tomographic images are treated, the noise reduction process can be performed without impairing the features of a structure of interest while preventing structures included in the tomographic images before and after the tomographic image of interest from being reflected in the tomographic image of interest after the processing.

Second, the present invention provides an image processing method comprising: an alignment step of sorting a pixel of interest and a plurality of adjacent pixels located around the pixel of interest in order of respective magnitudes of luminance values; a determination step of determining whether the pixel of interest is within a predetermined range intermediate in the pixel of interest and the plurality of adjacent pixels sorted in the alignment step; and a substitution step of, when the pixel of interest is not within the predetermined range, substituting the luminance value of the pixel of interest on the basis of the luminance value of a pixel within the predetermined range (Invention 5).

According to the above invention (Invention 5), rather than simply averaging the luminance value of the pixel of interest together with the luminance values of peripheral pixels (adjacent pixels located around the pixel of interest), the pixel of interest and the peripheral pixels are sorted in order of the magnitudes of luminance values, and when the pixel of interest is included in the predetermined intermediate range, no action is performed, while only when the pixel of interest is not included in the predetermined range, the luminance value of the pixel of interest is substituted based on the luminance value of a pixel within the predetermined range; therefore, only the noises in an image can be removed. As a result, the process of reducing noises can be performed without impairing the features such as the shape, brightness, and color of a characteristic structure captured in the image.

In the above invention (Invention 5), the predetermined range may preferably be set so as to include the same number of pixels before and after a pixel located at the center of the pixel of interest and the plurality of adjacent pixels sorted in the alignment step.

In the above invention (Invention 5, 6), the substitution step may preferably include substituting the luminance value of the pixel of interest with an average value of luminance values of pixels within the predetermined range (Invention 7) .

In the above invention (Invention 5 to 7), the pixel of interest may exist in an image that is one tomographic image of a plurality of successive tomographic images, and the plurality of adjacent pixels may be a plurality of adjacent pixels that are spatially located around the pixel of interest in the one tomographic image and tomographic images before and after the one tomographic image (Invention 8).

According to the above invention (Invention 8), even when successive tomographic images are treated, the noise reduction process can be performed without impairing the features of a structure of interest while preventing structures included in the tomographic images before and after the tomographic image of interest from being reflected in the tomographic image of interest after the processing.

Third, the present invention provides an image processing program for causing a computer to serve as the image processing device according to any one of Invention 1 to 4 or causing a computer to execute the image processing method according to any one of Invention 5 to 8 (Invention 9).

### [Advantageous Effect of the Invention]

According to the image processing device, image processing method, and image processing program of the present invention, it is possible to reduce noises in an image while maintaining the features of structures in the image.

### [Brief Description of Drawings]

FIG. 1 is a block diagram illustrating the overall configuration of an image processing device according to an embodiment of the present invention.
FIG. 2 is an explanatory diagram illustrating a state of acquiring the tomographic image of an ocular fundus by scanning the ocular fundus.
FIG. 3 is a set of explanatory diagrams schematically illustrating the relationship between a pixel of interest and adjacent pixels when the image processing device according to the present embodiment processes a single image.
FIG. 4 is a flowchart illustrating the flow of image processing in the present embodiment.
FIG. 5 is a set of explanatory diagrams schematically illustrating how the image processing device according to the present embodiment substitutes the luminance value of the pixel of interest.
FIG. 6 is a set of explanatory diagrams (part 1) schematically illustrating the relationship between a pixel of interest and adjacent pixels when the image processing device according to the present embodiment processes a plurality of successive tomographic images.
FIG. 7 is a set of explanatory diagrams (part 2) schematically illustrating the relationship between a pixel of interest and adjacent pixels when the image processing device according to the present embodiment processes a plurality of successive tomographic images.

### [Embodiments for Carrying out the Invention]

Hereinafter, one or more embodiments of the present invention will be described in detail with reference to the drawings. Here, the description will be made for an example of acquiring tomographic images of an ocular fundus E of the subject's eye by a tomography device and removing the noises from the tomographic images, but images to be processed in the present invention are not limited to the tomographic images of an ocular fundus, and the present invention can also be applied to cases in which other types of devices are used to capture images of other objects.

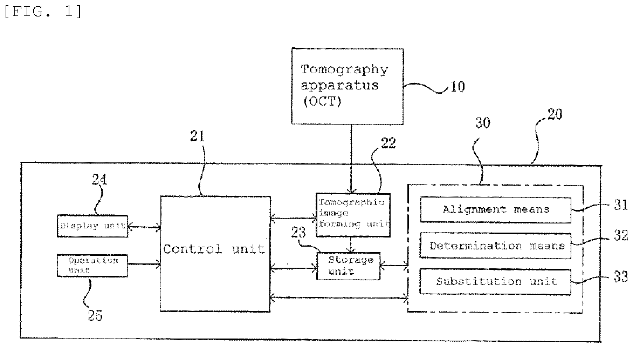

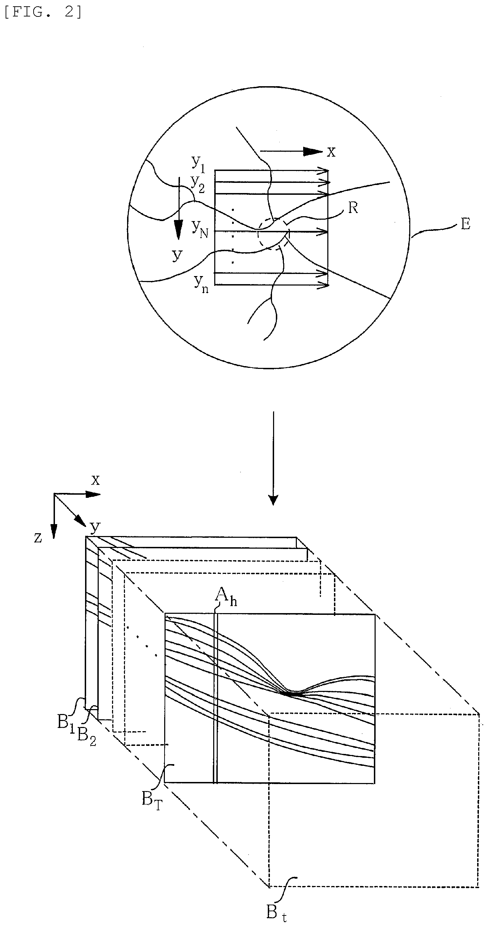

FIG. 1 is a block diagram illustrating the entire system which acquires tomographic images of the ocular fundus of a subject's eye and processes the images. This system includes a tomography apparatus 10. The tomography apparatus 10 may be an apparatus that captures tomographic pictures of the ocular fundus of a subject's eye using optical coherence tomography (OCT) and may operate, for example, in a Fourier-domain scheme. The tomography apparatus 10 is known in the art, so its detailed explanation will be omitted. The tomography apparatus 10 may be provided with a low-coherence light source, the light from which is split into reference light and signal light. As illustrated in FIG. 2, the signal light may be raster-scanned on an ocular fundus E, for example, in the X and Y directions. The signal light scanned and reflected from the ocular fundus E is superimposed with the reference light reflected from a reference mirror to generate interference light. On the basis of the interference light, OCT signals are generated which represent information in the depth direction (Z direction) of the ocular fundus.

The system further includes an image processing device 20. The image processing device 20 may have a control unit 21 that is realized by a computer composed of a CPU, a RAM, a ROM, and other necessary components. The control unit 21 executes an image processing program thereby to control the entire image processing. In addition, the image processing device 20 is provided with a tomographic image forming unit 22.

The tomographic image forming unit 22 may be realized by a dedicated electronic circuit that executes a known analyzing method, such as a Fourier-domain scheme, or realized by an image processing program that is executed by the previously described CPU. The tomographic image forming unit 22 may form tomographic images of the ocular fundus of a subject's eye on the basis of the OCT signals generated from the tomography apparatus 10.

For example, as illustrated in FIG. 2, when the ocular fundus E is scanned in the x direction at positions of y_{N} (N=1, * 2, ..., n) along the y direction, sampling is performed multiple times (m times) for each scan. Tomographic images (A-scan images) Aₕ (h=1, 2, ..., m) in the z direction are acquired at respective sampling points in the x direction, and tomographic images B_{N} (N=1, 2, ..., t) are formed from the A-scan images Aₕ. Each A-scan image is stored, for example, with a width of one pixel in the x direction and a length of n pixels in the z direction and, therefore, each tomographic image B_{N} is an image having a size of m×n pixels, which is also referred to as a B-scan image.

A plurality (t) of tomographic images B_{N} formed by the tomographic image forming unit 22 or a three-dimensional volume image assembled from the t tomographic images B_{N} may be stored in a storage unit 23 composed of a semiconductor memory, hard disk drive, or other appropriate storage device. The storage unit 23 may further store the above-described image processing program and other necessary programs and data.

The image processing device 20 is provided with an image processing unit 30. The image processing unit 30 may include an alignment means 31, a determination means 32, and a substitution means 33. As will be described later, the alignment means 31 sorts a pixel of interest in a processing target image and a plurality of adjacent pixels located around the pixel of interest in order of respective magnitudes of luminance values, the determination means 32 determines whether the pixel of interest is within a predetermined range intermediate in the pixel of interest and the plurality of adjacent pixels sorted by the alignment means, and when the pixel of interest is not within the predetermined range, the substitution means 33 substitutes the luminance value of the pixel of interest on the basis of the luminance value of a pixel within the predetermined range. Each means or each image processing in the image processing unit 30 may be realized by using a dedicated electronic circuit or by executing the image processing program.

A display unit 24 may be provided, which is, for example, composed of a display device such as an LCD. The display unit 24 may display tomographic images stored in the storage unit 23, images generated or processed by the image processing device 20, associated information such as information regarding the subject, and other information.

An operation unit 25 may be provided, which has, for example, a mouse, keyboard, operation pen, pointer, operation panel, and other appropriate components. The operation unit 25 may be used for selection of an image displayed on the display unit 24 or used for an operator to give an instruction to the image processing device 20 or the like.

The description will now be made for a flow of capturing tomographic pictures of the ocular fundus E of the subject's eye by the tomography apparatus 10 and removing noises in the tomographic images of the ocular fundus of the subject's eye, which are generated by the tomographic image forming unit 22 based on the captured tomographic pictures, through the image processing performed by the image processing unit 30. First, the flow of removing noises from a single processing target image B_{T} that is one of the obtained t tomographic images B_{N} will be described.

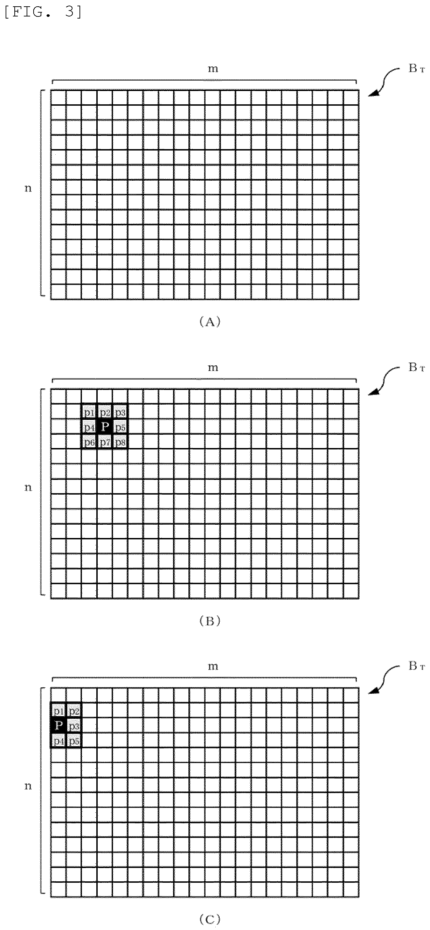

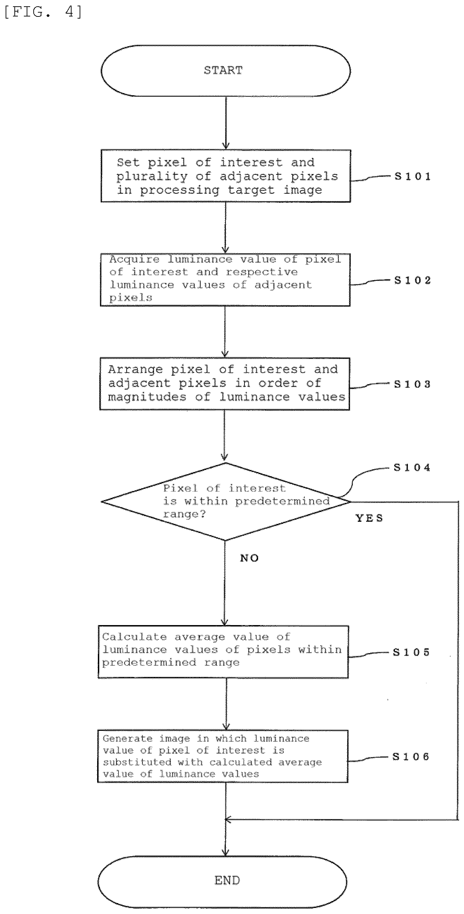

The processing target image B_{T} is an image having a size of m×n pixels, as illustrated in FIG. 3(A). FIG. 4 illustrates a flow of removing noises from the processing target image B_{T}. S101 to S106 in FIG. 4 correspond to steps 101 to 106 in the description of the flow, which will be described below. In the flow illustrated in FIG. 4, first, the alignment means 31 sets one pixel of interest in the processing target image B_{T} as a pixel of interest P and sets pixels located around the pixel of interest P, that is, a total of eight pixels: pixels located above and below and on the right and left of the pixel of interest P; and pixels located in contact with the four corners of the pixel of interest P, as adjacent pixels pᵢ (i=1 to 8) (step 101) . FIG. 3(B) illustrates the relationship between the pixel of interest P, which is set in the fourth column from the left and the third row from the top of the processing target image B_{T}, and the eight adjacent pixels pi located around the pixel of interest P.

When the pixel of interest P is set at an end of the processing target image B_{T}, eight adjacent pixels may not necessarily be set, and the number of adjacent pixels may be appropriately set within eight in accordance with the position of the pixel of interest. For example, when the pixel of interest is set in the first column from the left and the third row from the top of the processing target image B_{T}, as illustrated in FIG. 3(C), five pixels located above, below, diagonally right above, and diagonally right below and on the right of the pixel of interest P are set as the adjacent pixels.

The method of setting the adjacent pixels may be appropriately changed depending on the type of images to be processed, the purpose of noise removal, etc., such as setting only four pixels located above and below and on the right and left of the pixel of interest as the adjacent pixels or setting a total of 24 pixels: four pixels located above and below and on the right and left of the pixel of interest; four pixels located in contact with the four corners of the pixel of interest; and 16 pixels located further outside the eight pixels, as the adjacent pixels.

Subsequently, the alignment means 31 acquires a luminance value D of the pixel of interest P and luminance values di (i=1 to 8) of the eight adjacent pixels pi from the original image data of the processing target image B_{T} (step 102).

After acquiring the luminance value D of the pixel of interest P and the luminance values di of the adjacent pixels pᵢ, the alignment means 31 sorts the pixel of interest P and the adjacent pixels pi in order of magnitudes of the luminance values on the basis of the luminance value D of the pixel of interest P and the luminance value di of each adjacent pixel pi (step 103).

Subsequently, the determination means 32 determines whether the pixel of interest P is within a predetermined range intermediate in the pixel of interest P and the adjacent pixels pi sorted by the alignment means 31 (step 104). The predetermined range may be set so as to include the same number of pixels before and after a pixel located at the center of the pixel of interest P and the adjacent pixels pi sorted by the alignment means 31.

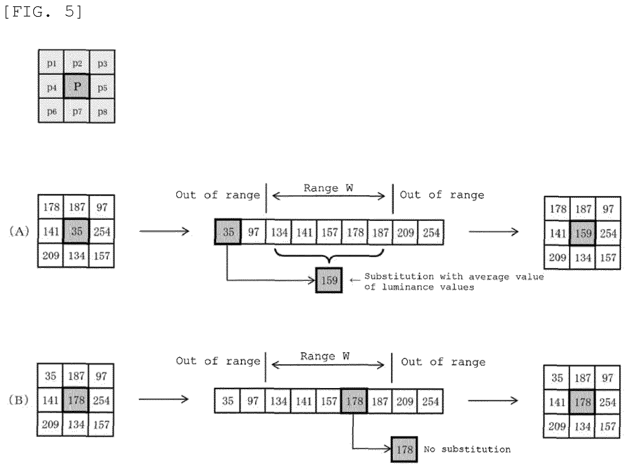

Specifically, as illustrated in FIG. 5(A), provided that the luminance value D of the pixel of interest P is 35 and the luminance values di of the adjacent pixels pi are 178, 187, 97, 141, 254, 209, 134, and 157 in this order, for example, when the pixel of interest P and the adjacent pixels pi are sorted from the left in ascending order of the luminance values, these pixels are sorted from the left in the order of the pixel of interest P, the adjacent pixel p₃, the adjacent pixel p₇, the adjacent pixel p₄, the adjacent pixel p₈, the adjacent pixel p₁, the adjacent pixel p₂, the adjacent pixel p₆, and the adjacent pixel p₅. Here, if the predetermined range is set to a range W composed of a total of five pixels including respective two pixels before and after a pixel, that is, the adjacent pixel p₈, located at the center of the pixel of interest P and the adjacent pixels pi sorted by the alignment means 31, the determination means 32 determines that the pixel of interest P is not within the range W. On the other hand, as illustrated in FIG. 5(B), provided that the luminance value D of the pixel of interest P and the luminance values di of the adjacent pixels pi are opposite to those in FIG. 5(A), that is, the luminance value D of the pixel of interest P is 178 and the luminance values di of the adjacent pixels pi are 35, 187, 97, 141, 254, 209, 134, and 157 in this order, when the pixel of interest P and the adjacent pixels pi are sorted from the left in ascending order of the luminance values, these pixels are sorted from the left in the order of the adjacent pixel pi, the adjacent pixel p₃, the adjacent pixel p₇, the adjacent pixel p₄, the adjacent pixel p₈, the pixel of interest P, the adjacent pixel p₂, the adjacent pixel p₆, and the adjacent pixel ps. In this case, the determination means 32 determines that the pixel of interest P is within the range W.

When the determination means 32 determines that the pixel of interest P is not within the range W, the substitution means 33 calculates the average value of the five pixels within the range W (step 105). Then, an image in which the luminance value of the pixel of interest P is substituted with the calculated average value is generated (step 106) and stored in the storage unit 23. For example, in the example illustrated in FIG. 5(A), the pixel of interest is outside the range W, and the average value of the five pixels within the range W is 159; therefore, an image in which the luminance value of the pixel of interest P is substituted with 159 is generated.

On the other hand, when the determination means 32 determines that the pixel of interest P is within the range W, the luminance value of the pixel of interest P is not substituted with the average value and remains without any change. For example, in the example illustrated in FIG. 5(B), the pixel of interest is within the range W, so the luminance value of the pixel of interest P remains 178.

In the present embodiment, the substitution means 33 substitutes the luminance value of the pixel of interest P with the average value of the luminance values of the five pixels within the predetermined range W, but the present invention is not limited to this, and depending on the quality of images and the properties (such as the shape, brightness, and size) of a structure to be extracted, for example, the luminance value of the pixel of interest P may be substituted with the luminance value of the pixel located at the center of the range W or may also be substituted based on the average value of the three center pixels within the range W.

The above-described process is repeated in the processing target image B_{T}, for example, while setting all the pixels each as the pixel of interest in order from the upper left pixel, and an image can be finally generated in which the noise removal process is performed on the entire processing target image B_{T}.

Thus, in the present invention, rather than simply averaging the luminance value of the pixel of interest in the processing target image together with the luminance values of the adjacent pixels located around the pixel of interest, the pixel of interest and the adjacent pixels located around the pixel of interest are sorted in order of the magnitudes of luminance values, and when the pixel of interest is included in the predetermined intermediate range, no action is performed, while only when the pixel of interest is not included in the predetermined range, the luminance value of the pixel of interest is substituted based on the luminance value of a pixel within the predetermined range. As a result, the process of reducing noises can be performed without impairing the features such as the shape, brightness, and color of a characteristic structure captured in the image.

In other words, if the pixel of interest is noise composed of a white point or a black point, the pixel of interest is highly likely to have a luminance value that is significantly different from the luminance values of the adjacent pixels located around the pixel of interest, and when the pixel of interest and the adjacent pixels are sorted in order of the magnitudes of luminance values, such a pixel having a luminance value deviating from the surrounding tendency will be located at an end of the arrangement. In the present invention, therefore, when the pixel of interest is within the predetermined range, it can be considered that the pixel of interest is not noise, so the luminance value of the pixel of interest is not substituted, while when the pixel of interest is not within the predetermined range, the pixel of interest is highly likely to be noise, so the luminance value of the pixel of interest is substituted with a luminance value that matches the surrounding tendency, and only the noises in the image can thereby be removed. As a result, the process of reducing noises can be performed without impairing the features such as the shape, brightness, and color of a characteristic structure captured in the image.

In the present embodiment, one of the tomographic images B_{N} acquired by the tomography apparatus 10 is used as the processing target image B_{T}, but the same image processing as the above can be performed using the plane image of an ocular fundus acquired by a scanning laser ophthalmoscope (SLO) as the processing target image to reduce the noises.

### <First Modified Example>

The flow of removing noises from a single processing target image B_{T} has been described hereinbefore, but the following description will be made for a method of setting the pixel of interest and the adjacent pixels using one of the t tomographic images B_{N}, which are obtained from spatially successive portions as illustrated in FIG. 2, as the processing target image B_{T} and also using a tomographic image B_{T-1} and a tomographic image B_{T+1} before and after the processing target image B_{T}. The tomographic image B_{T-1} and the tomographic image B_{T+1} are tomographic images successive to the processing target image B_{T} in the y direction illustrated in FIG. 2 and are images having a size of m×n pixels like the processing target image B_{T}. The tomography apparatus 10 and the image processing device 20 used for the image processing in the present modified example have the same configurations as those described above, so the detailed description will be omitted, but the tomographic image B_{T-1}, the processing target image B_{T}, and the tomographic image B_{T+1} are all stored in the storage unit 23.

In the present modified example, one pixel of interest in the processing target image B_{T} is set as a pixel of interest Q, and a total of 26 pixels that are spatially located around the pixel of interest in the processing target image B_{T} and in the tomographic image B_{T-1} and the tomographic image B_{T+1} before and after the processing target image B_{T} are set as adjacent pixels qᵢ (i=1 to 26).

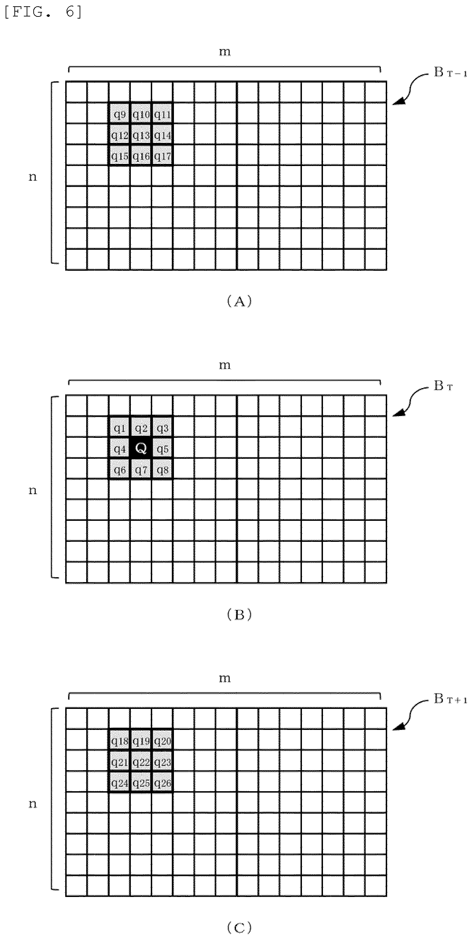

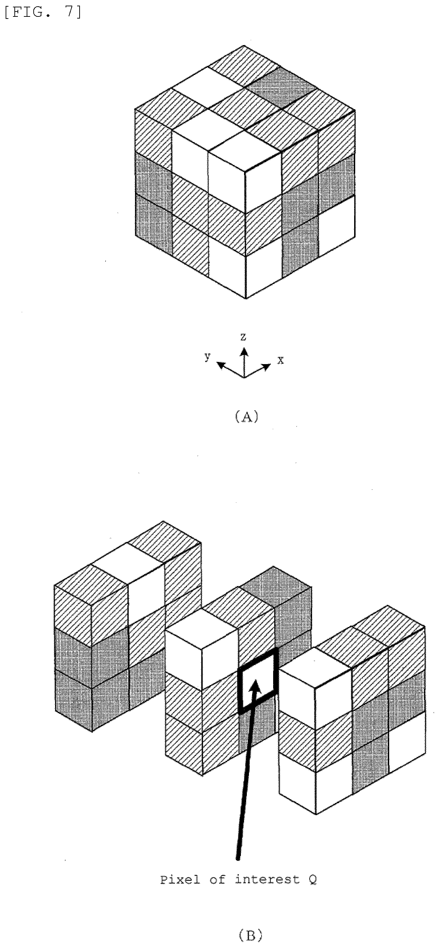

That is, the adjacent pixels qi in the present modified example are a total of 26 pixels including a total of eight pixels: pixels located above and below and on the right and left of the pixel of interest Q in the processing target image B_{T}; and pixels located in contact with the four corners of the pixel of interest and further including a total of 18 pixels that are spatially located around the pixel of interest Q when the tomographic image B_{T-1} and the tomographic image B_{T+1} are sorted side-by-side with the processing target image B_{T}. FIG. 6 illustrates the relationship between the pixel of interest Q, which is set in the fourth column from the left and the third row from the top of the processing target image B_{T}, and the 26 adjacent pixels qi which are specially located around the pixel of interest Q in the processing target image B_{T} and in the tomographic image B_{T-1} and the tomographic image B_{T+1} before and after the processing target image B_{T}. In addition, FIG. 7 illustrates a state of cutting out only the pixel of interest Q and the adjacent pixels qi from a state in which the processing target image B_{T} and the tomographic image B_{T-1} and the tomographic image B_{T+1} before and after the processing target image B_{T} are sorted side-by-side.

When the pixel of interest Q is set at an end of the processing target image B_{T}, 26 adjacent pixels may not necessarily be set, and the number of adjacent pixels may be appropriately set within 26 in accordance with the position of the pixel of interest. Also when no successive tomographic images exist before and after the processing target image, the number of adjacent pixels may be appropriately set within 26 in accordance with the position of the pixel of interest. The method of setting the adjacent pixels may be appropriately changed depending on the type of images to be processed, the purpose of noise removal, etc.

Also when the pixel of interest Q and the adjacent pixels qᵢ are set in this manner, noises can be removed by the image processing using the image processing unit 30 along the flow illustrated in FIG. 4. The flow of image processing is the same as the flow of removing noises from a single processing target image B_{T}, and the description will be omitted.

Thus, also when handling successive tomographic images, only the noises in the images can be removed by applying the image processing device and the image processing method according to the present embodiment. As a result, the noise reduction process can be performed without impairing the features such as the shape, brightness, and color of a characteristic structure captured in the images while preventing structures included in the tomographic images before and after the tomographic image of interest from being reflected in the tomographic image of interest after the processing.

### <Second Modified Example>

The tomographic images B_{N} acquired by the tomography apparatus 10 have been described as the processing targets hereinbefore, but it is also possible to perform image processing on a front image (En Face image) of a retinal blood vessel generated using OCT angiography (OCTA: Optical Coherence Tomography Angiography) to remove noises from the image. The OCT angiography is to generate an image such as an angiographic image without using a fluorescent agent, and the tomography apparatus 10 described in the above embodiments may be used to successively capture a plurality of images at the same site of the ocular fundus E of a subject's eye and generate the front image by regarding a change in the acquired time-difference tomographic images (B-scan images) of several ms as a blood flow change.

Specifically, a known change detection method such as an OMAG (Optical Microangiography) method or an SSADA (Split-spectrum Amplitude-decorrelation Angiography) method may be applied to a set of B-scan images obtained by successively capturing a plurality of images at the same site of the ocular fundus E of a subject's eye and generate one blood flow tomographic image. This operation may be repeated while changing the scan position in the y direction with respect to the observation target region, and one three-dimensional data set is generated from a plurality of successive blood flow tomographic images thus obtained. That is, the three-dimensional data set is the data obtained by stereoscopically modeling the observation target region of the ocular fundus E of the subject's eye.

When each B-scan image that constitutes the set of B-scan images is a tomographic image on the xz plane in FIG. 2, the blood flow tomographic images generated from the set of B-scan images are also tomographic images on the xz plane. From the three-dimensional data set, blood flow tomographic images may then be generated as a plurality of tomographic images on the xy plane that are successive in the z direction. The front image of the retinal blood vessel may be generated by multi-layering the plurality of blood flow tomographic images on the xy plane that are successive in the z direction.

If one of the plurality of blood flow tomographic images on the xy plane that are multi-layered is considered as the processing target image B_{T} in the first modified example and the blood flow tomographic images before and after the blood flow tomographic image overlapping each other so as to sandwich the blood flow tomographic image are considered as the tomographic image B_{T-1} and the tomographic image B_{T+1}, the same image processing method as that described in the above first modified example can be applied to the front image of the retinal blood vessel generated by the OCT angiography, and only the noises in the front image can be removed.

The image processing device and image processing method according to the present invention have been described above with reference to the drawings, but the present invention is not limited to the above embodiments, and various modifications can be carried out.

For example, for the processing target image B_{T}, not only the pixels included in the tomographic images B_{T-1} and B_{T+1} before and after the processing target image B_{T} but also the pixels included in tomographic images B_{T-2} and B_{T+2} further before and after the tomographic images B_{T-1} and B_{T+1} may be set as the adjacent pixels. Alternatively, the pixels included in the tomographic images B_{T-1} and B_{T+1} before and after the processing target image B_{T} may purposefully not be set as the adjacent pixels, and the adjacent pixels may be set using only the pixels included in the tomographic images B_{T}, B_{T-2}, and B_{T+2}. On the contrary, for simplification of the calculation, the adjacent pixels may be set only from the two images of the processing target image B_{T} and the tomographic image B_{T+1} which is adjacent to one side of the tomographic image B_{T}.

### [Industrial Applicability]

The present invention can be used as a method of efficiently removing noises in image diagnosis using an image with a poor signal-to-noise ratio (S/N) . In particular, in the ocular fundus image diagnosis and ocular fundus tomographic picture diagnosis in which it is difficult to use a powerful light source for the illumination light, there are many cases in which the shapes and number of microscopic structures in an image with poor S/N have to be analyzed, and it can therefore be expected that the present invention can be used as a powerful image noise reduction method.

### [Description of Reference Numerals]

- 10: Tomography apparatus
- 20: Image processing device
- 21: Control unit
- 22: Tomographic image forming unit
- 23: Storage unit
- 24: Display unit
- 25: Operation unit
- 30: Image processing unit
- 31: Alignment means
- 32: Determination means
- 33: Substitution unit

## Claims

1. An image processing device comprising:
an alignment means that arranges a pixel of interest and a plurality of adjacent pixels located around the pixel of interest in order of respective magnitudes of luminance values;
a determination means that determines whether the pixel of interest is within a predetermined range intermediate in the pixel of interest and the plurality of adjacent pixels sorted by the alignment means; and
a substitution means that, when the pixel of interest is not within the predetermined range, substitutes the luminance value of the pixel of interest on a basis of the luminance value of a pixel within the predetermined range.

2. The image processing device according to claim 1, wherein the predetermined range is set so as to include same number of pixels before and after a pixel located at center of the pixel of interest and the plurality of adjacent pixels sorted by the alignment means.

3. The image processing device according to claim 1 or 2, wherein the substitution means substitutes the luminance value of the pixel of interest with an average value of luminance values of pixels within the predetermined range.

4. The image processing device according to any one of claims 1 to 3, wherein
the pixel of interest exists in an image that is one tomographic image of a plurality of successive tomographic images, and
the plurality of adjacent pixels is a plurality of adjacent pixels that are spatially located around the pixel of interest in the one tomographic image and tomographic images before and after the one tomographic image.

5. An image processing method comprising:
an alignment step of sorting a pixel of interest and a plurality of adjacent pixels located around the pixel of interest in order of respective magnitudes of luminance values;
a determination step of determining whether the pixel of interest is within a predetermined range intermediate in the pixel of interest and the plurality of adjacent pixels sorted in the alignment step; and
a substitution step of, when the pixel of interest is not within the predetermined range, substituting the luminance value of the pixel of interest on a basis of the luminance value of a pixel within the predetermined range.

6. The image processing method according to claim 5, wherein the predetermined range is set so as to include same number of pixels before and after a pixel located at center of the pixel of interest and the plurality of adjacent pixels sorted in the alignment step.

7. The image processing method according to claim 5 or 6, wherein the substitution step includes substituting the luminance value of the pixel of interest with an average value of luminance values of pixels within the predetermined range.

8. The image processing method according to any one of claims 5 to 7, wherein
the pixel of interest exists in an image that is one tomographic image of a plurality of successive tomographic images, and
the plurality of adjacent pixels is a plurality of adjacent pixels that are spatially located around the pixel of interest in the one tomographic image and tomographic images before and after the one tomographic image.

9. An image processing program for causing a computer to serve as the image processing device according to any one of claims 1 to 4 or causing a computer to execute the image processing method according to any one of claims 5 to 8.
